# EUROPEAN PATENT SPECIFICATION

(11) **EP 0 386 882 B1**
(45) Date of publication and mention of the grant of the patent: **14.09.1994**
(21) Application number: 90301212.8
(22) Date of filing: 06.02.1990
(51) Int. Cl.: C12N 15/86, C12N 15/49, C12N 5/10, A61K 39/395, A61K 39/21, A61K 48/00

(54) **Packaging defective HIV provirus, cell lines, and uses thereof**
Verpackungsdefizientes HIV-Provirus, Zellinien und deren Verwendung
Provirus d'HIV défectif pour l'empaquetage, lignées cellulaires et leur utilisation

(30) Priority: 06.02.1989 US 307664
(43) Date of publication of application: 12.09.1990
(73) Proprietor: DANA FARBER CANCER INSTITUTE, Boston Massachusetts 02115 (US)
(72) Inventor: Sodroski, Joseph, Medford, MA 02155 (US); Haseltine, William A., Cambridge, MA 02140 (US); Gottlinger, Heinrich, D-8000 München 71 (DE); Lever, Andrew, London SW17 0RE (GB)
(74) Representative: Davies, Jonathan Mark

(56) References cited:
- EP-A- 0 273 782
- EP-A- 0 334 301
- WO-A-87/06258
- WO-A-89/05349
- JOURNAL OF THEORETICAL BIOLOGY, vol. 130, 1988, pages 469-480, Academic Press Ltd; J.C. SANFORD: "Applying the PDR principle to AIDS
- JOURNAL OF AIDS, 4, p. 317-323
- JOURNAL OF VIROLOGY 62, p. 1120-1124

## Description

The present invention is directed to vectors comprising a packaging defective HIV provirus, and the use of these vectors to create HIV packaging defective cell lines, and the uses thereof. Most preferably, the HIV provirus is an HIV-1 provirus.

The human immunodeficiency virus (HIV-I, also referred to as HTLV-III, LAV or HTLV-III/LAV) is the etiological agent of the acquired immune deficiency syndrome (AIDS) and related disorders [Barre-Sinoussi, et al., Science 220:868-871 (1983); Gallo et al, Science 224:500-503 (1984); Levy et al., Science 225:840-842 (1984); Popovic et al., Science 224:497-500 (1984); Sarngadharan et al., Science 224:506-508 (1984); Siegal et al., N. Engl. J. Med. 305:1439-1444 (1981)]. The disease is characterized by a long asymptomatic period followed by progressive degeneration of the immune system and the central nervous system. Studies of the virus indicate that replication is highly regulated, and both latent and lytic infection of the CD4 positive helper subset of T-lymphocytes occur in tissue culture [Zagury et al., Science 231:850-853 (1986)]. The expression of the virus in infected patients also appears to be regulated to enable evasion of the immune response. Molecular studies of the regulation and genomic organization of HIV-I show that it encodes a number of genes [Ratner et al., Nature 313:277-284 (1985); Sanchez-Pescador et al., Science 227:484-492 (1985); Muesing et al., Nature 313:450-457 (1985); Wain-Hobson et al., Cell 40:9-17 (1985)].

Retroviruses are typically classified as belonging to one of three subfamilies, namely oncoviruses, spumaviruses and lentiviruses. Infection by oncoviruses is typically associated with malignant disorders. These viruses typically contain a single-stranded, plus-strand RNA genome of approximately 8,000 to 10,000 nucleotides encompassing the gag, pol and env, genes, as well as long terminal repeat (LTR) sequences. Some of the oncoviruses contain oncogenes. It is generally believed that spumaviruses are not pathogenic in vivo, although they induce foamy cytopathic changes in tissue culture. Infection by lentiviruses is generally slow and causes chronic debilitating diseases after a long latency period. These viruses, in addition to the gag, pol, and env genes possess a number of additional genes with regulatory functions.

The human immunodeficiency viruses (HIV) has been classified as a lentivirus, because it too causes slow infection and has structural properties in common with such viruses. [See Haase, A.T., Nature 322: 130-136 (1986)].

HIV-1 shares the gag, pro, pol, and env genes, respectively with other retroviruses [Haseltine, W.A., Journal of Acquired Immune Deficiency Syndrome, 1:217-240 (1988)]. HIV-1 also possesses additional genes modulating viral replication. The HIV-1 genome encodes vif, vpr, tat, rev, vpu and nef proteins [Haseltine, W.A., Journal of Acquired Immune Deficiency Syndrome, supra]. Additionally, the long terminal repeats (LTRs) of HIV contain cis-acting sequences that are important for integration, transcription and polyadenylation. Additional cis-acting signals allow regulation of HIV sequences by some of the novel HIV gene products, (Haseltine, W.A., Journal of Acquired Immune Deficiency Syndrome, supra). Sodroski et al., Science 231: 1549-1553 (1986); Arya et al., Science 229:69-73 (1985); Sodroski et al., Science 227:171-173 (1985); Sodroski et al., Nature 321:412-417 (1986); Feinberg et al., Cell 46:807-817 (1986) Wong-Staal et al, AIDS Res. and Human Retroviruses 3: 33-39 (1987); which are all incorporated herein by reference]. The region between the 5′ major splice donor and the gag gene initiation codon is highly conserved in different HIV-1 strains sequenced to date [Myers, G., et al, Theoretical Biology and Biophysics, (1988)].

Most of these genes encode products that are necessary for the viral life cycle. For example, the tat gene encodes a 14kD protein that is critical for HIV replication and gene expression [Rosen, C.A., et al., Nature 319:555-559 (1986); Sodroski, J. et al., Science 227:171-173 (1985); Arya et al, Science 229:supra, Sodroski, et al., Science 229, supra and Dayton, A., et al., Cell 44:941-497 (1986) which are all incorporated herein by reference]. Another gene necessary for replication is the rev gene. [Sodroski, et al., Nature 321:412-417 (1986), which is both incorporated herein by reference].

In some oncoviruses, cis-acting sequences located between the 5′, LTR and the gag gene initiation codon have been located which are necessary for the efficient packaging of the viral RNA into virions [Bender, M.A., et al, J. Virol 61:1639-1646 (1987), Katz, R.A., et al, J. Virol 59:163-167 (1986), Mann, R., et al, Cell 33:153-159 (1983), Pugatsch, T., et al, Virology 128:505-511 (1983), Watanabe, S., et al, Proc. Natl. Acad. Sci. 79:5986-5990 (1983) Eglitis, M.A., et al, Bio Techniques 6:608-614 (1988) which are incorporated herein by reference]. In addition to these sequences, sequences overlapping the gag gene were found to contribute to the efficiency of viral RNA encapsidation by Moloney murine leukemia virus [Adam, M.A., et al, J. Virol. 62:3802-3806 (1988)]. The signals needed for packaging of lentiviruses RNA, (such as HIV RNA) into virion particles have not been identified.

Although a great deal of research has been expended on understanding HIV-1, the life cycle of this retrovirus is not completely understood.

In addition, a great deal of research has been directed to developing a vaccine to the virus, but there have been no reports of success to date. This is, in part, due to the lack of conservation in the antigenically active parts of the virus and in part because the functionally important regions of viral proteins and/or inactivated viral particles are poorly immunogenic.

Accordingly, it would be extremely useful to have a provirus that produced HIV proteins but which was not lethal because the viral RNA could not be packaged into virions. Using this packaging-defective provirus vector, it would be possible to create packaging defective cell lines that could be used to investigate the packaging mechanism of the virus and to develop strategies to interfere with this packaging mechanism. Significantly, the virions produced by such packaging negative proviruses could be used for vaccines and as a system for efficiently introducing a desired gene into a mammalian cell.

### Summary of the Invention

We have now discovered a vector comprising
(a) nucleotides corresponding to an
   HIV genome to encode HIV gag, env, and pol proteins, referred to as the HIV segment, wherein the HIV gag, env, and pol proteins expressed by the vector in combination are capable of forming an HIV virion, and said vector contains a deletion of the HIV packaging segment necessary to package HIV RNA, which is located between the 5' major splice donor and the initiation codon of the gag gene; and
(b) a promoter operably linked to the HIV segment;
wherein said HIV proteins expressed by the vector are capable of forming HIV virions that do not contain sufficient HIV RNA to result in a replication competent HIV virus. Preferably the HIV packaging segment corresponds to a segment just downstream of the 5' major splice donor, and about 14 bases upstream of the gag initiation codon. In one embodiment it is a 19 base segment having the sequence AAAAATTTTGACTAGCGGA.

This vector can be used to transform a preselected cell line to result in an HIV packaging defective cell line. Preferably, one would transform a cell line using at least two vectors, which collectively contain the HIV nucleotides necessary to express HIV gag, pol, and env products, but wherein each vector by itself does not contain the HIV nucleotides necessary to express all three products. In addition, each vector does not have a sufficient number of nucleotides corresponding to nucleotides of the HIV genome between the 5' major splice donor and the gag gene to package HIV RNA. Preferably, each vector would contain a different marker gene. The transformed cell line would express HIV virions but would not be able to package HIV RNA into these virions. Thus, these virions could be used to generate antibodies, as a vaccine or as a method of transferring a desired gene product to a different cell line capable of infection by HIV.

### Summary of the Drawings

Figure 1 is a schematic of the HIV-1 genome from the 5' LTR to the gag initiation codon showing the 5' major splice donor (SD) and the site of the deletion in a vector representing one embodiment of this invention, pHXBΔP1.

Figure 2a is an autoradiogram of the immunoprecipitation of ³⁵S-labelled viral protein from COS-1 cells with AIDS patient serum.

Figure 2b is an electron micrograph of COS-1 cells transfected with pHXBΔP1 showing virion particles of normal HIV-1 morphology.

Figure 3 is an autoradiogram of immunoprecipitation of labelled viral proteins from Jurkat T cell lysates or supernatants exposed to supernatants from COS-1 cells that were transfected or mock transfected.

Figure 4 is an RNA dot blot test.

### Detailed Description of the Invention

We have now discovered that it is possible to make HIV packaging defective vectors and cell lines. We have found that the region between the 5' major splice donor and the gag gene initiation codon in HIV viruses contains sequences necessary for packaging of HIV RNA into virions. One can prepare a vector comprising a packaging defective HIV provirus wherein the vector contains a nucleotide sequence which corresponds to a sufficient number of nucleotides from an HIV genome to express desired HIV products, but contains a deletion of the HIV packaging segment necessary to package HIV RNA, which is located in the region between the 5' major splice donor and the gag gene initiation codon.

These sequences preferably correspond to the genome of HIV-1, HIV-2 and simian immunodeficiency virus (SIV). [See Ratner, et al, Nature 313, supra, Sanches-Pescador et al, Science 227, supra, Muesing, et al, Nature 313, supra, Wain-Hobson et al, Cell 40, supra, Guyader, M. et al, Nature 326:662-669 (1987); Chakrabarti et al, Nature 328:543-547 (1987) and Hirsch, V., et al, Cell 49:307-319 (1987) which are all incorporated herein by reference].

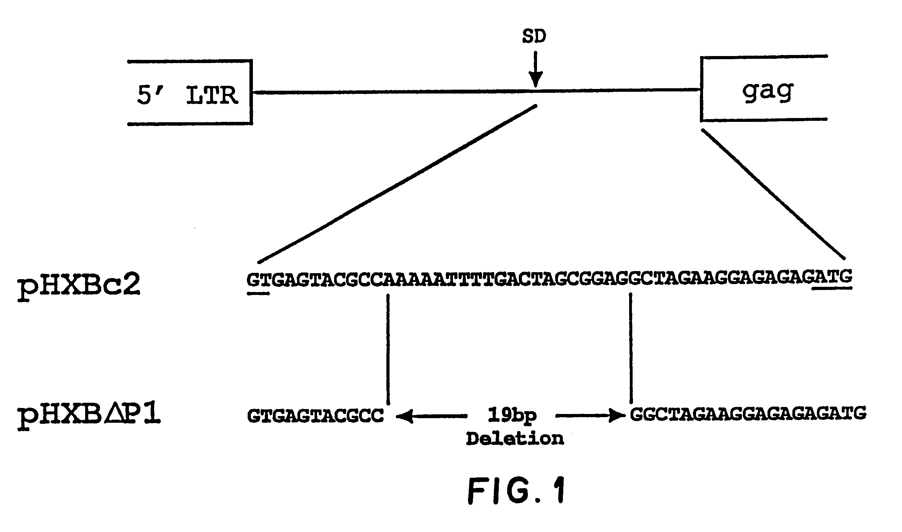

Preferably, the vector contains a deletion of the HIV packaging sequence corresponding to the segment immediately downstream of the 5' major splice donor and just upstream of the gag gene initiation codon. Typically, the vector could contain nucleotides ranging from about 14 bases to 2 bases upstream of the gag initiation codon; for example either the 14 upstream bases or 5 upstream bases and still be packaging deficient. In one embodiment the vector contains a deletion of a nucleotide sequence beginning about 9 bases downstream of the 5' major splice donor and continuing to about 14 bases upstream of the gag initiation codon. The number of bases that need to be left out can vary greatly, for example, the 19 base pair deletion AAAAATTTTGACTAGCGGA deletion in HIV-1 is sufficient to result in loss of packaging ability (See Figure 1). However, even smaller deletions in this region should also result in loss of packaging efficiencies. Indeed, it is expected that a deletion as small as about 5 base pairs in this region should remove packaging ability. Thus the size of a particular deletion can readily be determined based upon the present disclosure by the person of ordinary skill in the art.

The vector should contain an HIV nucleotide segment containing a sufficient number of nucleotides corresponding to nucleotides of the HIV genome to express functional HIV gene products, but as aforesaid, should not contain a sufficient number of nucleotides corresponding to the region between the 5′ major splice donor and the gag gene initiation codon to permit efficient packaging of the viral RNA into virions. In using these vectors to establish HIV packaging defective cell lines it is preferred that such cell lines do not produce any infectious HIV. Although a cell line transformed by these packaging deficient vectors would have low infectivity because the cells are packaging defective, some RNA can still be packaged into the virion. Accordingly, it is preferable that the HIV nucleotide segment does not correspond to the entire HIV genome so that if some of the viral RNA is packaged into the virion, what is packaged will not be a replication competent virus.

Preferably, one would want to have at least two different vectors, each containing a different portion of the HIV genome and also not containing the sequence necessary for viral packaging. Then by co-transfecting a cell with each vector the cell would still be able to express all the HIV structural proteins and produce virions. In one preferred embodiment the vector would not contain sequences corresponding to an HIV LTR but would contain sequences corresponding to a promoter region and/or another genome's polyadenyltation sequences. Selection of particular promoters and polyadenylaton sequences can readily be determined based upon the particular host cell.

In one preferred embodiment one vector would include sequences permitting expression of HIV proteins upstream of env and the second vector would permit expression of the remaining proteins. For example, one vector would contain an HIV nucleotide segment corresponding to a sufficient number of nucleotides upstream of the gag initiation codon to the env gene sequence to express the 5′-most gene products. The other vector would contain an HIV nucleotide segment corresponding to a sufficient number of nucleotides downstream of the gag gene sequence and including a functional env gene sequence. Such vectors can be chemically synthesized from the reported sequences of the HIV genomes or derived from the many available HIV proviruses, by taking advantage of the known restriction endonuclease sites in these viruses by the skilled artisan based upon the present disclosure. Preferably, one would also add a different marker gene to each vector, i.e., co-transfect a preselected cell line with these different vectors and by looking for a cell containing both markers, one would have a cell line that has co-transfected with the two vectors. Such a cell would be able to produce all of the HIV proteins. However, although virions would be produced, the RNA corresponding to the entire viral sequences would not be packaged in these virions. One can use more than two vectors if desired, e.g. a gag-pol vector, an env vector and a vif/vpu vector.

Virtually any cell line can be used. Preferably, one would use a mammalian cell line, for example, CV-1, Hela, Raji, RD, SW480 or CHO cell lines.

In order to increase production of the viral cellular products, one could use a different promoter than the 5′ LTR, i.e., replace the 5′ LTR with a promoter that will preferentially express genes under its control in a particular cell line. For example, the CMV promoter will preferably express genes in CV-1 or Hela cells. The particular promoter used can be readily determined by the person of ordinary skill in the art, based upon the particular host cell line to be used.

In order to increase the level of viral cellular products one can also add enhancer sequences to the vector to get enhancement of the HIV LTR and/or promoter. Particular enhancer sequences can readily be determined by the person of ordinary skill in the art depending upon the host cell line.

One can also add vectors that express viral enhancer proteins, such as those of herpes virus, hepatitis B virus, which act on HIV LTRs to enhance the level of virus product, or cellular transactivator proteins. Cellular transactivation proteins include NF κ-B, UV light responsive factors and other T cell activation factors well known to the person of ordinary skill in the art.

By using a series of vectors that together would contain the complete HIV genome, one can create cell lines that produce a virion that is identical to the HIV virion, except that the virion does not contain the HIV RNA. The virions can readily be obtained from these cells. For example, the cells would be cultured and supernatant collected. Depending upon the desired use the supernatant containing the virions can be used or these virions can be separated from the supernatant by standard techniques. Typically, this would include gradiant centrifugation, filtering, etc.

These attenuated virions would be extremely useful in preparing a vaccine. The virions can be used to generate an antigenic response to the HIV virions and because these virions are identical to the actual HIV virions, except that the interior of these virions do not contain the viral RNA, the vaccine created should be particularly useful.

These virions can also be used to raise antibodies to the virion that can then be used for a variety of purposes, e.g. screening for the virion, developing target system for the virions, etc.

Additionally, these HIV packaging deficient cell lines can be extremely useful as a means of introducing a desired gene, for example, a heterologous gene into mammalian cells.

These virions could be used as an extremely efficient way to package desired genetic sequences into target cells infectable by HIV. This would be done by preparing a vector containing a nucleotide segment containing nucleotides corresponding to the packaging nucleotides of the HIV virus (HIV packaging region), a predetermined gene, and flanking the packaging sequence and the predetermined gene with sequences corresponding to a sufficient number of sequences from HIV LTRs for packaging, reverse transcription, integration and gene expression. The packaging region used would preferably correspond to at least the region between the 5' major splice donor and just upstream of the gag initiation codon, more preferably the region between the 5' major splice donor and the Bal I site in the gag gene. When this vector is used to transfect one of the HIV packaging deficient cells, it is the nucleotide sequence from this vector that will be packaged in the virions. These "HIV packaged" genes could then be targeted to cells infectable by HIV. This method of transformation is expected to be much more efficient than current methods. Further, by appropriate choice of genes, one could also monitor the method of HIV infection.

Additionally, these HIV packaging defective cell lines can be used to study various stages of the HIV life cycle, both in vivo and in vitro systems by a system because the cells will express HIV cellular proteins, but will not package the RNA.

The present invention is further illustrated by the following examples. These examples are provided to aid in the understanding of the invention and are not to be construed as limitation thereof.

The region between the HIV-1 5' LTR and the gag gene is shown in Figure 1 which shows the 5' major splice donor (SD) and site of deletion in a vector described below, pHXBΔP1. A 19 base-pair deletion in this region was created in an infectious HIV-1 proviral clone contained on the plasmid pHXBc2 of Fisher, A.G., et al, Nature 316: 262-265 (1985). This plasmid also contains an SV40 origin of replication to allow efficient gene expression in COS-1 cells. The mutation was produced by the site-directed mutagenesis as described in Kunkel, T.A., et al, Methods in Enzymology 154, 367-382 (1987), and the sequence confirmed by DNA sequencing (Sanger, F., et al, Proc. Natl. Acad. Sci. 74:5463-5467 (1977). The mutated plasmid was designated pHXBΔP1.

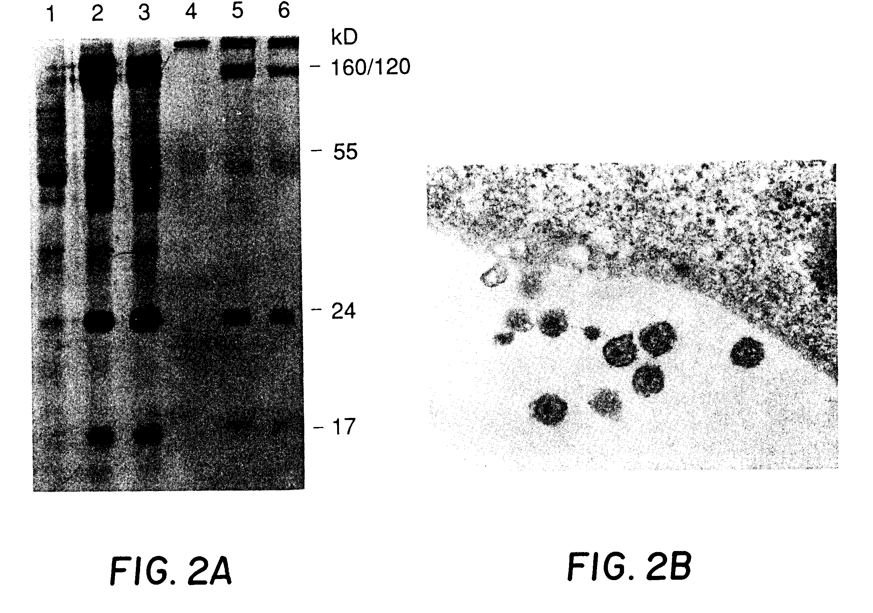

To evaluate the effect of the mutation on viral protein expression and virion production, COS-1 cells were transfected with the pHXBc2 and pHXBΔP1 plasmids by the DEAE-dextran procedure [Lopata et al, Nucl. Acids Res. 12:5707-5717 (1984); Queen and Baltimore, Cell 33:741-748 (1983); (Sodroski, J., et al, Science 231:1549-1553 (1986) which are incorporated herein by reference]. COS-1 cell lysates and supernatants radiolabelled with ³⁵S-cysteine Sodroski, J., et al, Science 231, supra) at 48 hours after transfection were precipitated with 19501 AIDS patient serum. The overall level of viral protein detected in cell lysates was comparable for the vectors containing the wild-type HXBc2 and the HIV packaging defective HXBΔP1. See Figure 2A, which shows immunoprecipitation of ³⁵S-labelled viral proteins from COS-1 cell lysates (lanes 1-3) or supernatants (lanes 4-6) with 19501 patient serum, after transfection with no DNA (lanes 1 and 4), 10µg pHXBc2 (lanes 2 and 5) or 10µg pHXBΔP1 (lanes 3 and 6). The overall level of viral protein detected in cell lysates was comparable for cells transfected by either HXBc2 or HXBΔP1. The level of viral proteins precipitated from the supernatants of COS-1 cells was slightly less with HXBΔP1 than with HXBc2. The amount of reverse transcriptase (RT) activity measured in the supernatants of COS-1 cell transfected by pHXBΔP1 was 60% of that measured in cells transfected with the HXBc2 vector (data not shown). COS-1 cells transfected with pHXBΔP1 were fixed 48 hours following transfection and examined by electron microscopy. Viral particles, including budding forms, of normal HIV-1 morphology were observed. Figure 2B is an electron micrographs of COS-1 cells transfected with PHXBΔP1 showing virus particles of normal HIV-1 morphology.

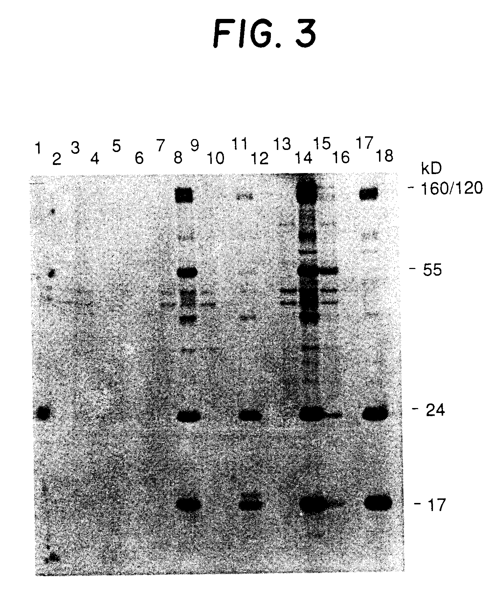

To evaluate the effect of the HXBΔP1 mutation on HIV-1 replication, supernatants from COS-1 cells transfected with pHXBc2 and pHXBΔP1 were filtered (0.2µ) and RT measured. Supernatants containing equal amounts of RT activity of mutant and wild-type viruses were added to Jurkat human T lymphocytes. The Jurkat cultures along with a mock-infected culture were maintained with medium changes every three days. At intervals aliquots of Jurkat cells were labelled and assessed for expression of HIV-1 proteins by immunoprecipitation with 19501 AIDS patient serum. Figure 3 shows immunoprecipitation of labelled viral proteins from Jurkat T cell lysates (lanes 1-3, 7-9 and 13-15) or supernatants (lanes 4-6, 10-12 and 16-18) exposed to supernatants from COS-1 cells that were mock transfected (lanes 1, 4, 7, 10, 13 and 16), pHXBc2 (lanes 2, 5, 8, 11, 14 and 17), or transfected with pHXBΔP1 (lanes 3, 6, 9, 12, 15 and 18). The Jurkat cells were examined at day 7 (lanes 1-6), day 14 (lanes 7-12) and day 21 (13-18) following infection. Jurkat cultures exposed to HXBΔP1 exhibited marked delays in and lower levels of viral protein production relative to those exposed to pHXBc2, the wild-type virus. Virus replication in human T lymphocytes transfected by HXBΔP1 is thus seen to be significantly attenuated compared with cells transfected by HXBc2.

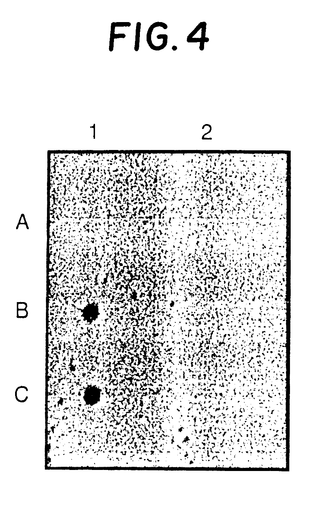

Supernatants from the above cultures were 0.2µ-filtered and equivalent amounts of reverse transcriptase activity pelleted by centrifugation at 12000xg for one hour at 20°C. Viral pellets were lysed by NP40 in the presence of vanadyl ribonucleotides and dilutions of virus dot-blotted onto nitrocellulose filters. Some samples were treated with sodium hydroxide (5M at 60°C for 15 minutes) prior to dot-blotting. Filters were hybridized with a DNA probe consisting of HIV-1 gag and env gene sequences, washed and autoradiographed as previously described in Maniatis, T., et al, Molecular Cloning, Cold Spring Harbor Laboratory, (1982). For the wild-type HXBc2 virus, a signal specific for RNA could be detected after blotting 1000 reverse transcriptase units of filtered supernatant (not shown). For the HXBΔP1, even 5 X 10⁴ reverse transcriptase units of supernatant gave no detectable signal. Figure 4 is an RNA dot blot without (column 1) and with (column 2) sodium hydroxide treatment following blotting of filtered supernatants from the Jurkat cultures. The supernatants contained a reverse transcriptase activity of 5 x 10⁴ cpm of HXBΔP1 (row A), 5 x 10⁴ cpm of HXBc2 (row B), or 1 x 10⁵ cpm of HXBc2 (row C). These results indicate that the efficiency of packaging virus-specific RNA into virions for cells transfected with a packaging defective viral vector according to the present invention is less than 2% of the wild-type virus.

The results indicate that the region between the 5′ LTR and gag gene of HIV-1 is important for packaging viral RNA into virions. A mutation in this region exhibits minimal effects on the ability of the provirus to produce proteins and virion particles following transfection, but markedly decreases the level of virion RNA and attenuates virus replication in a human CD4-positive lymphocyte line. HIV-1 replicates in cultured CD4-positive cells via cell-free transmission and cell-to-cell transmission, the latter involving the contact of infected and uninfected cells [Fisher, A.G., et al, Nature 316:262-265 (1985), Sodroski, J., et al, Science 231:1549-1553 (1986), Strebel, K., et al, Nature 328:728-730 (1987)].

## Claims

1. A vector comprising
(a) nucleotides corresponding to an
HIV genome to encode HIV gag, env, and pol proteins, referred to as the HIV segment, wherein the HIV gag, env, and pol proteins expressed by the vector in combination are capable of forming an HIV virion, and said vector contains a deletion of the HIV packaging segment necessary to package HIV RNA, which is located between the 5' major splice donor and the initiation codon of the gag gene; and
(b) a promoter operably linked to the HIV segment;
wherein said HIV proteins expressed by the vector are capable of forming HIV virions that do not contain sufficient HIV RNA to result in a replication competent HIV virus.

2. A vector system comprising at least two vectors, referred to as a first vector and second vector, wherein the vectors of said system comprises:
(a) nucleotides corresponding to an HIV genome to encode HIV gag, env, and pol proteins, referred to as the HIV segment, wherein the HIV gag, env and pol proteins expressed by the vectors in combination are capable of forming an HIV virion, said vectors contain a deletion of the HIV packaging segment necessary to package HIV RNA, which is located between the 5' major splice donor and the initiation codon of the gag gene necessary to effectively package HIV RNA, and said vectors contain a deletion whereby said HIV gag, env, and pol proteins cannot be encoded by a single vector; and
(b) a promoter operably linked to the HIV segment;
wherein said vector system is capable of forming virions that do not contain sufficient HIV RNA to result in a replication competent virus.

3. The vectors of claims 1 or 2, wherein the promoter is an HIV LTR.

4. The vectors of claims 1 or 2, wherein the promoter is a promoter that will preferentially express gene products in particular cells.

5. The vectors of claim 4, wherein the promoter is a CMV promoter.

6. The vectors of claims 1 or 2, wherein the deletion of the HIV packaging segment is a nucleotide sequence downstream of the 5' major splice donor to about 5 bases upstream of the gag gene initiation codon.

7. The vectors of claims 1 or 2, wherein the deletion of the HIV packaging segment is a nucleotide sequence beginning about 9 bases downstream of the 5' major splice donor to about 14 bases upstream of the gag gene initiation codon.

8. The vectors of claims 1 or 2, wherein the deletion of the HIV packaging segment is the sequence AAAAATTTTGACTAGCGGA.

9. The vectors of claims 1 or 2, wherein the HIV genome is selected from the group consisting of HIV-1, HIV-2 and SIV.

10. The vectors of claims 1 or 2, wherein the HIV genome is the HIV-1 genome.

11. The vector of claim 8, wherein the HIV genome is the HIV-1 genome.

12. An HIV packaging defective cell line which comprises a preselected cell line transformed by the vectors of claims 1 or 2.

13. A method of producing an HIV packaging defective cell line which comprises:
(a) transforming a preselected cell line with the vectors of claim 1 or 2.

14. The method of claim 13, wherein the preselected cell line is a mammalian cell.

15. The method of claim 13, wherein the cell is transformed by the two vectors which collectively are capable of expressing HIV proteins to form the virion.

16. The method of claim 15, wherein the cell is transformed by two vectors and one vector contains a nucleotide sequence from just upstream of the gag initiation codon to just upstream of the env gene sequence, and the second vector contains a nucleotide sequence downstream of the gag gene sequence and including a functional env sequence.

17. The method of claim 15, wherein each vector contains a different marker gene sequence.

18. A stable cell line transformed by the method of claim 15.

19. A method of producing a vaccine to HIV which comprises culturing the cell line of claim 18, and collecting virions produced from the cell line to use as a vaccine.

20. A method of transferring genes to mammalian cells which comprises:
(a) transfecting the cell line of claim 18 with a vector containing a nucleotide sequence corresponding to a preselected gene downstream of a nucleotide sequence corresponding to an HIV packaging sequence to package HIV RNA, and wherein the preselected gene and the HIV packaging sequence are flanked on each side with a sequence corresponding to a sufficient number of nucleotides corresponding to HIV LTRs to be packaged by the HIV packaging sequence,
wherein the HIV packaging sequence and the HIV LTR sequences correspond to the same HIV genome as the HIV nucleotides;
(b) culturing the cell line transformed in step (a);
(c) collecting virions produced from the cell line of step (b); and
(d) contacting the virions of step (c) with a predetermined target mammalian cell in vitro.

21. The method of claim 20, wherein the nucleotides or the HIV packaging sequence are the nucleotides from the 5' major splice donor to the Bal I site in the gag gene and the HIV LTR sequences correspond to the entire HIV LTRs.

## Patentansprüche

1. Vektor, umfassend
(a) einem für HIV gag-, env- und pol-Proteine codierenden HIV-Genom entsprechende Nucleotide, als das HIV-Segment bezeichnet, wobei die von dem Vektor in Kombination exprimierten HIV gag-, env- und pol-Proteine in der Lage sind, ein HIV-Virion zu bilden und der Vektor eine Deletion des Verpackungssegments enthält, das zur Verpackung der HIV-RNA notwendig ist, die zwischen dem 5' Hauptspleißdonor und dem Initiationscodon des gag-Gens angeordnet ist; und
(b) einen mit dem HIV-Segment wirkverbundenen Promoter;
wobei die von dem Vektor exprimierten HIV-Proteine in der Lage sind, HIV-Virionen zu bilden, die nicht ausreichend HIV-RNA enthalten, um in einem replikationsfähigen HIV-Virus zu resultieren.

2. Vektorsystem, umfassend wenigstens zwei Vektoren, die als erster Vektor und als zweiter Vektor bezeichnet werden, wobei die Vektoren des Systems
(a) einem für HIV gag-, env- und pol-Proteine codierenden HIV-Genom entsprechende Nucleotide, als das HIV-Segment bezeichnet, wobei die von den Vektoren in Kombination exprimierten HIV gag-, env- und pol-Proteine in der Lage sind, ein HIV-Virion zu bilden, die Vektoren eine Deletion des Verpackungssegments enthalten, das zur wirksamen Verpackung der HIV-RNA notwendig ist, die zwischen dem 5' Hauptspleißdonor und dem Initiationscodon des gag-Gens angeordnet ist, und die Vektoren eine Deletion enthalten, wodurch die HIV gag-, env-und pol-Proteine nicht durch einen einzelnen Vektor codiert werden können; und
einen mit dem HIV-Segment wirkverbundenen Promoter umfassen; wobei das Vektorsystem in der Lage ist, Virionen zu bilden, die nicht ausreichend HIV-RNA enthalten, um in einem replikationsfähigen HIV-Virus zu resultieren.

3. Vektoren gemäß Anspruch 1 oder 2, wobei der Promoter eine HIV LTR ist.

4. Vektoren gemäß Anspruch 1 oder 2, wobei der Promoter ein Promoter ist, der bevorzugt Genprodukte in bestimmten Zellen exprimiert.

5. Vektoren gemäß Anspruch 1 oder 2, wobei der Promoter ein CMV-Promoter ist.

6. Vektoren gemäß Anspruch 1 oder 2, wobei die Deletion des HIV-Verpackungssegments eine Nukleotidsequenz stromabwärts des 5' Hauptspleißdonors bis etwa 5 Basen stromaufwärts des gag-Gen Initiationscodons ist.

7. Vektoren gemäß Anspruch 1 oder 2, wobei die Deletion des HIV-Verpackungssegments eine Nukleotidsequenz ist, die etwa 9 Basen stromabwärts des 5' Hauptspleißdonors bis etwa 14 Basen stromaufwärts des gag-Gen Initiationscodons beginnt.

8. Vektoren gemäß Anspruch 1 oder 2, wobei die Deletion des HIV-Verpackungssegments die Sequenz AAAAATTTTGACTAGCGGA ist.

9. Vektoren gemäß Anspruch 1 oder 2, wobei das HIV-Genom aus der aus HIV-1, HIV-2 und SIV bestehenden Gruppe ausgewählt ist.

10. Vektoren gemäß Anspruch 1 oder 2, wobei das HIV-Genom das HIV-1-Genom ist.

11. Vektor gemäß Anspruch 8, wobei das HIV-Genom das HIV-1-Genom ist.

12. Zellinie mit einem HIV-Verpackungsdefekt, die eine durch die Vektoren gemäß Anspruch 1 oder 2 transformierte, vorausgewählte Zellinie umfaßt.

13. Verfahren zur Herstellung einer Zellinie mit einem HIV-Verpackungsdefekt, das
(a) die Transformation einer vorausgewählten Zellinie mit den Vektoren gemäß Anspruch 1 oder 2
umfaßt.

14. Verfahren gemäß Anspruch 13, wobei die vorausgewählte Zelle eine Säugetierzelle ist.

15. Verfahren gemäß Anspruch 13, wobei die Zelle von den beiden Vektoren transformiert wird, die gemeinsam in der Lage sind, HIV-Proteine zur Bildung des Virions zu exprimieren.

16. Verfahren gemäß Anspruch 15, wobei die Zelle durch zwei Vektoren transformiert wird und ein Vektor eine Nukleotidsequenz von unmittelbar stromaufwärts des gag-Initiationscodons bis unmittelbar stromaufwärts der env-Gensequenz enthält und der zweite Vektor eine Nukleotidsequenz stromabwärts der gag-Gensequenz enthält und eine funktionelle env-Gensequenz einschließt.

17. Verfahren gemäß Anspruch 15, wobei jeder Vektor eine unterschiedliche Marker-Gensequenz enthält.

18. Stabile Zellinie, transformiert durch das Verfahren gemäß Anspruch 15.

19. Verfahren zur Herstellung eines Impfstoffes für HIV, das das Züchten der Zellinie gemäß Anspruch 18 und das Sammeln von von der Zellinie produzierten Virionen zur Verwendung als Impfstoff umfaßt.

20. Verfahren zur Übertragung von Genen auf Säugetierzellen, umfassend:
(a) Transfizieren der Zellinie gemäß Anspruch 18 mit einem Vektor, der eine Nukleotidsequenz enthält, die einem vorausgewählten Gen stromabwärts einer einer HIV-Verpackungssequenz zur Verpackung von HIV-RNA entsprechenden Nukleotidsequenz entspricht, und wobei das vorausgewählte Gen und die HIV-Verpackungssequenz auf beiden Seiten von einer Sequenz flankiert sind, die einer ausreichenden Anzahl von HIV LTRs entsprechenden Nukleotiden entspricht, um von der HIV-Verpackungssequenz verpackt zu werden, wobei die HIV-Verpackungssequenz und die HIV LTR-Sequenzen demselben HIV-Genom wie die HIV-Nukleotide entsprechen;
(b) Zuchten der in Schritt (a) transformierten Zellinie;
(c) Sammeln von von der Zellinie gemäß Schritt (b) produzierten Virionen; und
(d) Kontaktieren der Virionen gemäß Schritt (c) mit einer vorbestimmten Zielsäugetierzelle in vitro.

21. Verfahren gemäß Anspruch 20, wobei die Nukleotide der HIV-Verpackungssequenz die Nukleotide von 5' Hauptspleißdonor zur Bal I Stelle im gag-Gen sind und die HIV LTR-Sequenzen den gesamten HIV LTRs entsprechen.

## Revendications

1. Vecteur comprenant
(a) des nucléotides correspondant à un génome de HIV destiné au codage des protéines gag, env et pol de HIV, désigné sous le nom de segment de HIV, les protéines gag, env et pol de HIV exprimées par le vecteur de manière associée étant capables de former un virion HIV, et ledit vecteur contenant une délétion du segment d'encapsidation de HIV nécessaire à l'encapsidation de l'ARN de HIV, qui est située entre le donneur d'épissure principal en 5' et le codon d'initiation du gène gag, et
(b) un promoteur lié de manière fonctionnelle au segment de HIV ;
lesdites protéines de HIV exprimées par le vecteur étant capables de former des virions HIV qui ne contiennent pas de quantités d'ARN de HIV suffisantes pour qu'il en résulte un virus HIV présentant une compétence de réplication.

2. Système de vecteurs comprenant au moins deux vecteurs, désignés sous les noms de premier vecteur et second vecteur, dans lequel les vecteurs comprennent :
(a) des nucléotides correspondant à un génome de HIV destiné au codage des protéines gag, env et pol de HIV, désigné sous le nom de segment de HIV, les protéines gag, env et pol de HIV exprimées par les vecteurs de manière associée étant capables de former un virion HIV, et lesdits vecteurs contenant une délétion du segment d'encapsidation de HIV nécessaire à l'encapsidation de l'ARN de HIV, qui est située entre le donneur d'épissure principal en 5' et le codon d'initiation du gène gag nécessaire à l'encapsidation efficace de l'ARN de HIV, et lesdits vecteurs contenant une délétion rendant impossible le codage desdites protéines gag, env et pol de HIV par un seul vecteur; et
(b) un promoteur lié de manière fonctionnelle au segment de HIV ;
ledit système de vecteurs étant capable de former des virions qui ne contiennent pas une quantité d'ARN de HIV suffisante pour qu'il en résulte un virus présentant une compétence de réplication.

3. Vecteurs suivant la revendication 1 ou 2, dans lesquels le promoteur est une longue répétition terminale de HIV.

4. Vecteurs suivant la revendication 1 ou 2, dans lesquels le promoteur est un promoteur qui exprime préférentiellement des produits de gènes dans des cellules particulières.

5. Vecteurs suivant la revendication 4, dans lesquels le promoteur est un promoteur de CMV.

6. Vecteurs suivant la revendication 1 ou 2, dans lesquels la délétion du segment d'encapsidation de HIV est une séquence de nucléotides en aval du donneur d'épissure principal en 5' jusqu'à environ 5 bases en amont du codon d'initiation du gène gag.

7. Vecteurs suivant la revendication 1 ou 2, dans lesquels la délétion du segment d'encapsidation de HIV consiste en une séquence de nucléotides commençant environ 9 bases en aval du donneur d'épissure principal en 5' jusqu'à environ 14 bases en amont du codon d'initiation du gène gag.

8. Vecteurs suivant la revendication 1 ou 2, dans lesquels la délétion du segment d'encapsidation de HIV consiste en la séquence AAAAATTTTGACTAGCGGA.

9. Vecteurs suivant la revendication 1 ou 2, dans lesquels le génome de HIV est choisi dans le groupe consistant en les génomes de HIV-1, HIV-2 et SIV.

10. Vecteurs suivant la revendication 1 ou 2, dans lesquels le génome de HIV est le génome de HIV-1.

11. Vecteur suivant la revendication 8, dans lequel le génome de HIV est le génome de HIV-1.

12. Lignée cellulaire présentant une défectivité d'encapsidation de HIV, qui consiste en une lignée cellulaire présélectionnée transformée par les vecteurs suivant la revendication 1 ou 2.

13. Procédé de production d'une lignée cellulaire présentant une défectivité d'encapsidation de HIV, qui comprend :
(a) la transformation d'une lignée cellulaire présélectionnée avec les vecteurs suivant la revendication 1 ou 2.

14. Procédé suivant la revendication 13, dans lequel la lignée cellulaire présélectionnée est une lignée de cellules de mammifère.

15. Procédé suivant la revendication 13, dans lequel la cellule est transformée par les deux vecteurs qui, collectivement, sont capables d'exprimer les protéines de HIV pour former le virion.

16. Procédé suivant la revendication 15, dans lequel la cellule est transformée par deux vecteurs et un vecteur contient une séquence de nucléotides allant d'un site juste en amont du codon d'initiation gag à un site juste en amont de la séquence du gène env, et le second vecteur contient une séquence de nucléotides en aval de la séquence du gène gag et comprenant une séquence env fonctionnelle.

17. Procédé suivant la revendication 15, dans lequel chaque vecteur contient une séquence de gène marqueur différente.

18. Lignée cellulaire stable transformée par le procédé suivant la revendication 15.

19. Procédé de production d'un vaccin contre le HIV, qui comprend la culture de la lignée cellulaire suivant la revendication 18 et la collecte des virions produits à partir de la lignée cellulaire pour l'utilisation comme vaccin.

20. Procédé de transfert de gènes à des cellules de mammifère, qui comprend :
(a) la transfection de la lignée cellulaire suivant la revendication 18 avec un vecteur contenant une séquence de nucléotides correspond à un gène choisi préalablement en aval d'une séquence de nucléotides correspondant à une séquence d'encapsidation de HIV pour l'encapsidation de l'ARN de HIV, le gène choisi préalablement et la séquence d'encapsidation de HIV étant flanqués de chaque côté d'une séquence correspondant à un nombre suffisant de nucléotides correspondant à des longues répétitions terminales de HIV pour l'encapsidation par la séquence d'encapsidation de HIV, la séquence d'encapsidation de HIV et les séquences consistant en longues répétitions terminales de HIV correspondant au même génome de HIV que les nucléotides de HIV ;
(b) la culture de la lignée cellulaire transformée dans l'étape (a) ;
(c) la collecte des virions produits à partir de la lignée cellulaire de l'étape (b) ; et
(d) la mise en contact des virions de l'étape (c) avec une cellule cible prédéterminée de mammifère, in vitro.

21. Procédé suivant la revendication 20, dans lequel les nucléotides de la séquence d'encapsidation de HIV sont les nucléotides allant du donneur d'épissure principal en 5' jusqu'au site Bal I dans le gène gag et les séquences consistant en longues répétitions terminales de HIV correspondent à la totalité des longues répétitions terminales de HIV.
